(19) Europäisches Patentamt / European Patent Office / Office européen des brevets

(11) **EP 4 133 999 A1**

(12) **EUROPEAN PATENT APPLICATION**
published in accordance with Art. 153(4) EPC

(43) Date of publication:
**15.02.2023 Bulletin 2023/07**

(21) Application number: **20934385.4**

(22) Date of filing: **08.05.2020**

(51) International Patent Classification (IPC):
**A61B 5/0215** (1990.01)    **A61M 25/01** (1990.01)

(52) Cooperative Patent Classification (CPC):
**A61B 5/0215; A61M 25/01**

(86) International application number:
**PCT/CN2020/089285**

(87) International publication number:
**WO 2021/223248 (11.11.2021 Gazette 2021/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Insight Lifetech Co., Ltd.**
**Shenzhen, Guangdong 518101 (CN)**

(72) Inventors:
• **LIN, Jiayan**
  **Shenzhen, Guangdong 518101 (CN)**
• **ZHENG, Yuxiao**
  **Shenzhen, Guangdong 518101 (CN)**
• **SHAO, Xiaohu**
  **Shenzhen, Guangdong 518101 (CN)**
• **LI, Lianbo**
  **Shenzhen, Guangdong 518101 (CN)**
• **SONG, Liang**
  **Shenzhen, Guangdong 518101 (CN)**
• **CHEN, Lili**
  **Shenzhen, Guangdong 518101 (CN)**

(74) Representative: **Isern Patentes y Marcas S.L.**
**Avda. Diagonal, 463 Bis, 2°**
**08036 Barcelona (ES)**

(54) **SYSTEM AND METHOD FOR TRACKING CARDIAC CIRCULATORY EVENT BY USING BLOOD PRESSURE**

(57) Disclosed is a system for tracking a cardiac circulatory event by using a blood pressure. The system comprises: a pressure measurement apparatus configured to respectively measure the pressure of a side in a blood vessel that is close to a proximal side and the pressure of a side in the blood vessel that is away from the proximal side and generate a first pressure signal and a second pressure signal; and a host connected to the pressure measurement apparatus and configured to receive the first pressure signal and the second pressure signal, wherein the host distinguishes each of all cardiac cycles on the basis of the first pressure signal and the second pressure signal and calculates the ratio of a pressure value of the second pressure signal to a pressure value of the first pressure signal corresponding to the pressure value of the second pressure signal to obtain a pressure ratio, and generates a target pressure waveform; and the host obtains a target derivative waveform on the basis of the target pressure waveform, and determines a target period in any cardiac cycle on the basis of the target derivative waveform, thereby obtaining the average value of corresponding pressure ratios in the target time, wherein the target period is the time, in the cardiac cycle, when a derivative value of the target derivative waveform changes within a preset value range.

EP 4 133 999 A1

Start

Measuring the pressure of a side close to a proximal side in a blood vessel at a certain sampling rate to obtain a first pressure signal, and measuring the pressure of a side away from the proximal side in the blood vessel to obtain a second pressure signal — S10

Distinguishing each of all cardiac cycles according to the first pressure signal and the second pressure signal — S20

Synchronizing the first pressure signal and the second pressure signal so that a moment when a peak value of the first pressure signal appears is the same as a moment when a peak value of the second pressure signal appears in the cardiac cycle, and rejecting invalid signals in the first pressure signal and the second pressure signal — S30

Calculating a ratio of a pressure value of the second pressure signal to a pressure value, corresponding to the pressure value of the second pressure signal, of the first pressure signal according to the first pressure signal and the second pressure signal to obtain a pressure ratio, thereby obtaining a target pressure waveform; and obtaining a derivative value of the pressure ratio with respect to time according to the target pressure waveform, thereby obtaining a target derivative waveform — S40

Determining a target period in any cardiac cycle according to the target derivative waveform, wherein the target period is a period in the cardiac cycle when a derivative value of the target derivative waveform changes within a preset value range — S50

Obtaining an average value of corresponding pressure ratios in the target period — S60

End

FIG. 9

## Description

## BACKGROUND OF INVENTION

## Field of Invention

The present disclosure particularly relates to a system and a method for tracking a cardiac circulatory event by using a blood pressure.

## Description of Related Art

As coronary artery disease is one of the major causes of death worldwide, the ability to diagnose more effectively, monitor and treat coronary artery disease can save lives. Coronary angiography is a conventional technology used to evaluate stenotic lesions of coronary arteries, but coronary angiography cannot reflect the real situation of a coronary vascular function, so it is basically impossible to determine whether coronary arteries of stenotic lesions are related to the symptom of myocardial ischemia of a patient or not. Currently, a method used to judge the stenotic lesion of coronary artery in clinical practice is mainly a technology of Fractional Flow Reserve (FFR for short) obtained by pressure guide wire examination.

However, the following defects exist in the process of obtaining the FFR: maximum congestion inducing drugs (e.g., adenosine, adenosine triphosphate, ATP) are required to be injected into the coronary artery prior to measuring the FFR so that the coronary artery is in a state of maximum congestion, and this process may prolong the clinical operation time, bring discomfort to patients, significantly increase the medical cost, and also cause allergic reaction to patients.

## SUMMARY OF INVENTION

The present disclosure is made in view of the above-mentioned situation and its object is to provide a system and a method for tracking a cardiac circulatory event by using a blood pressure, with which it is unnecessary to inject maximum congestion inducing drugs.

For this purpose, a first aspect of the present disclosure provides a system for tracking a cardiac circulatory event by using a blood pressure, characterized by including: a pressure measurement apparatus configured to measure a pressure of a side close to a proximal side in a blood vessel at a certain sampling rate to generate a first pressure signal, and measure a pressure of a side away from the proximal side in the blood vessel to generate a second pressure signal; and a host connected to the pressure measurement apparatus and configured to receive the first pressure signal and the second pressure signal, the host distinguishes each of all cardiac cycles on the basis of the first pressure signal and the second pressure signal and calculates a ratio of a pressure value of the second pressure signal to a pressure value of the first pressure signal corresponding to the pressure value of the second pressure signal to obtain a pressure ratio and generate a target pressure waveform, the host obtains a derivative value of the pressure ratio with respect to time on the basis of the target pressure waveform, thereby obtaining a target derivative waveform, and the host determines a target period in any cardiac cycle on the basis of the target derivative waveform, thereby obtaining an average value of corresponding pressure ratios in the target period, wherein the target period is a period in the cardiac cycle when a derivative value of the target derivative waveform changes within a preset value range.

In the present disclosure, the pressure measurement apparatus is configured to measure, at a certain sampling rate, the pressure of the side, close to the proximal side, in the blood vessel to generate the first pressure signal, and measure the pressure of the side, away from the proximal side, in the blood vessel to generate the second pressure signal, and the host may be connected to the pressure measurement apparatus and configured to receive the first pressure signal and the second pressure signal. The host obtains the ratio of the pressure value of the second pressure signal to the pressure value, corresponding to the pressure value of the second pressure signal, of the first pressure signal on the basis of the first pressure signal and the second pressure signal to obtain a pressure ratio and generate a target pressure waveform. The host obtains the derivative value of the pressure ratio with respect to time on the basis of the target pressure waveform, thereby obtaining the target derivative waveform. The host determines the target period in any cardiac cycle on the basis of the target derivative waveform, thereby obtaining the average value of corresponding pressure ratios in the target period, wherein the target period is a period, in the cardiac cycle, when the derivative value of the target derivative waveform changes within the preset value range. In this case, with the present disclosure, the pathological condition of a patient's blood vessel can be determined without the injection of the maximum congestion inducing drugs.

In the system for tracking the cardiac circulatory events by using the blood pressure involved by the first aspect of the present disclosure, optionally, the host obtains a first pressure waveform at least including a complete cardiac cycle on the basis of the first pressure signal, , the host obtains a second pressure waveform at least including a complete cardiac cycle on the basis of the second pressure signal, and the host distinguishes each of all the cardiac cycles on the basis of the first pressure waveform and/or the second pressure waveform. Therefore, the first pressure waveform and the second pressure waveform can be obtained, and the host can distinguish all the cardiac cycles on the basis of the first pressure waveform and/or the second pressure waveform.

In the system for tracking the cardiac circulatory event by using the blood pressure involved by the first aspect of the present disclosure, optionally, the host is configured to synchronize the first pressure waveform and the second pressure waveform so that a moment when a peak value of the first pressure waveform appears is the same as a moment when a peak value of the second pressure waveform appears in the cardiac cycle. Therefore, the first pressure waveform and the second pressure waveform can be synchronized, which facilitates the subsequent obtaining of an accurate pressure ratio.

In the system for tracking the cardiac circulatory events by using the blood pressure involved by the first aspect of the present disclosure, optionally, on the basis of the target pressure waveform, the host obtains target periods respectively corresponding to multiple cardiac cycles and calculates average values of corresponding pressure ratios in the target periods of all the cardiac cycles, thereby averaging the multiple obtained average values to obtain a target average value. Therefore, the target average value can be obtained.

In the system for tracking the cardiac circulatory event by using the blood pressure involved by the first aspect of the present disclosure, optionally, the cardiac cycle includes a systolic phase of systole and a diastolic phase of diastole, and the target period is within the diastolic phase. Therefore, the target periods within the diastolic phase can be obtained.

In the system for tracking the cardiac circulatory event by using the blood pressure involved by the first aspect of the present disclosure, optionally, the host preprocesses the first pressure signal and the second pressure signal to reject invalid signals in the first pressure signal and the second pressure signal, and the host filters the first pressure signal and the second pressure signal. Therefore, it is facilitated to subsequently obtain a more accurate target period.

In the system for tracking the cardiac circulatory event by using the blood pressure involved by the first aspect of the present disclosure, optionally, the invalid signals include signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal. Therefore, the invalid signals in the first pressure signal and the second pressure signal can be rejected.

In the system for tracking the cardiac circulatory events by using the blood pressure involved by the first aspect of the present disclosure, optionally, the target period is continuous, and the time duration of the target periods is greater than a preset time value. Therefore, a more valid target period can be obtained.

A second aspect of the present disclosure provides a method for tracking a cardiac circulatory event by using a blood pressure includes: at a certain sampling rate measuring the pressure of a side close to a proximal side in a blood vessel to obtain a first pressure signal, and meanwhile, measuring the pressure of a side away from the proximal side in the blood vessel to obtain a second pressure signal; distinguishing each of all cardiac cycles according to the first pressure signal and the second pressure signal; synchronizing the first pressure signal and the second pressure signal so that a moment when a peak value of the first pressure signal appears is the same as a moment when a peak value of the second pressure signal appears in the cardiac cycle, and rejecting invalid signals in the first pressure signal and the second pressure signal; calculating a ratio of a pressure value of the second pressure signal to a pressure value corresponding to the pressure value of the second pressure signal of the first pressure signal according to the first pressure signal and the second pressure signal to obtain a pressure ratio, thereby obtaining a target pressure waveform; obtaining a derivative value of the pressure ratio with respect to time according to the target pressure waveform, thereby obtaining a target derivative waveform; determining a target period in any cardiac cycle according to the target derivative waveform, thereby obtaining an average value of corresponding pressure ratios in the target period, wherein the target period is a period, in the cardiac cycle, when a derivative value of the target derivative waveform changes within a preset value range, wherein the invalid signals include signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal.

In the present disclosure, the pressure of the side close to the proximal side in the blood vessel is measured at a certain sampling rate to generate the first pressure signal, and the pressure of the side away from the proximal side in the blood vessel is measured to generate the second pressure signal. All the cardiac cycles are distinguished according to the first pressure signal and the second pressure signal; the first pressure signal and the second pressure signal are synchronized, and the invalid signals in the first pressure signal and the second pressure signal are rejected; the ratio of the pressure value of the second pressure signal to the pressure value corresponding to the pressure value of the second pressure signal of the first pressure signal is calculated according to the first pressure signal and the second pressure signal to obtain the pressure ratio, thereby obtaining the target pressure waveform; the derivative value of the pressure ratio with respect to time is obtained according to the target pressure waveform, thereby obtaining the target derivative waveform; the target period in any cardiac cycle is determined according to the target derivative waveform, wherein the target period is a period, in the cardiac cycle, when the derivative value of the target derivative waveform changes within the preset value range; and the average value of the corresponding pressure ratios in the target period is obtained. In this case, according to the present disclosure, the pathological condition of a patient's blood vessel can be determined without the injection of the maximum congestion inducing drugs.

In the method for tracking the cardiac circulatory event by using the blood pressure involved by the second aspect of the present disclosure, optionally, the cardiac cycle includes a systolic phase of systole and a diastolic phase of diastole; the target period is continuous and is within the diastolic phase, and the time duration of the target period is greater than a preset time value; and target periods respectively corresponding to multiple cardiac cycles are obtained according to the target pressure waveform, and average values of corresponding pressure ratios in the target periods of all the cardiac cycles are calculated, thereby averaging the multiple obtained average values to obtain a target average value. In this case, a more valid target period can be obtained.

According to the present disclosure, a system and a method for tracking a cardiac circulatory event by using a blood pressure are provided, with which it is unnecessary to inject maximum congestion inducing drugs.

## BRIEF DESCRIPTION OF THE DRAWINGS

Fig. 1 is a structural block diagram showing a system for tracking a cardiac circulatory event by using a blood pressure involved by an example of the present disclosure;

Fig. 2 is a schematic diagram showing a system for tracking a cardiac circulatory event by using a blood pressure involved by an example of the present disclosure;

Fig. 3 is a schematic diagram showing that a system for tracking a cardiac circulatory event by using a blood pressure involved by an example of the present disclosure is intervened in a human body;

Fig. 4 is blood vessel images of a preset part involved by an example of the present disclosure;

Fig. 5 is a sectional diagram showing that a system involved by an example of the present disclosure is applied to blood vessels;

Fig. 6 is a pressure waveform diagram showing multiple cardiac cycles involved by an example of the present disclosure;

Fig. 7 is a pressure waveform diagram showing multiple cardiac cycles involved by an example of the present disclosure;

Fig. 8 is a pressure waveform diagram showing multiple cardiac cycles involved by another example of the present disclosure; and

Fig. 9 is a schematic flow chart showing a method for tracking a cardiac circulatory event by using a blood pressure involved by an example of the present disclosure.

## DETAILED DESCRIPTION

Hereinafter, preferred implementations of the present disclosure will be described in detail with reference to the accompanying drawings. In the following description, the same components are given the same symbols, and repeated description is omitted. In addition, the accompanying drawings are merely schematic, and the proportions of the dimensions of the components relative to each other or the shapes of the components, etc. may differ from the reality.

In addition, reference to subheadings etc. in the following description of the present disclosure is not intended to limit the content or scope of the present disclosure, but is merely intended to serve as a reminder for reading. Such subheadings are neither understood to be used to segment the content of the article, nor should the content under the subheadings be limited only to the scope thereof.

The present disclosure provides a system for tracking a cardiac circulatory event by using a blood pressure, in which the pathological condition of a patient's blood vessel can be determined without the injection of the maximum congestion inducing drugs.

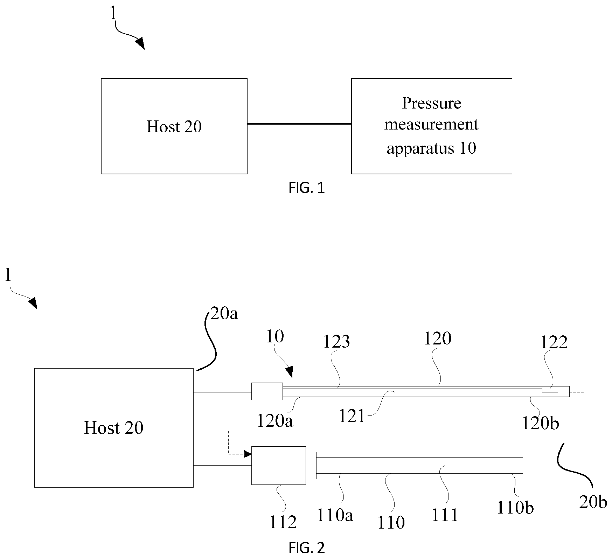

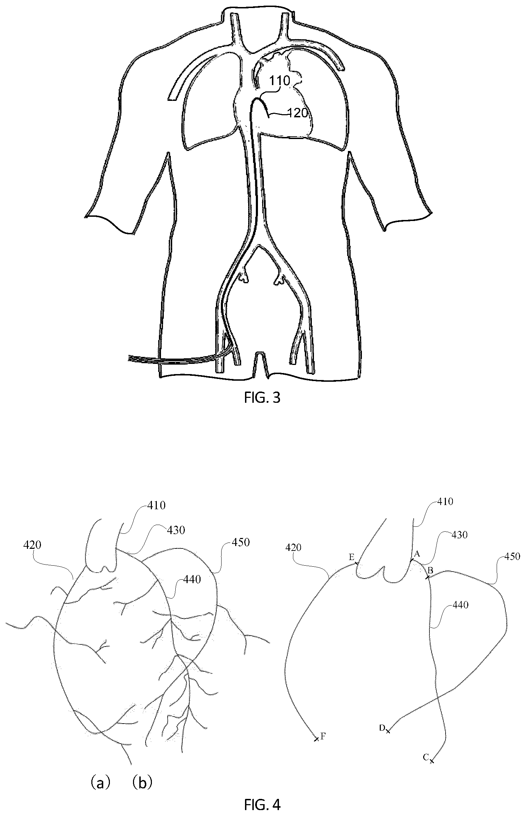

Fig. 1 is a structural block diagram showing a system 1 for tracking a cardiac circulatory event by using a blood pressure involved by an example of the present disclosure. Fig. 2 is a schematic diagram showing a system 1 for tracking a cardiac circulatory event by using a blood pressure involved by an example of the present disclosure. Fig. 3 is a schematic diagram showing that a system 1 for tracking a cardiac circulatory event by using a blood pressure involved by an example of the present disclosure is intervened in a human body.

In some examples, as shown in Fig. 1 and Fig. 2, the system 1 ("system 1" for short) for tracking the cardiac circulatory event by using the blood pressure may include a pressure measurement apparatus 10 and a host 20. The pressure measurement apparatus 10 may be connected to the host 20. The pressure measurement apparatus 10 may measure the pressure in a blood vessel (also referred to as "intravascular pressure") and generate a pressure signal, after which the pressure signal may be transmitted to the host 20 for processing.

In some examples, as shown in Fig. 1 and Fig.

2, the side close to the host 20 (described later) may be a proximal side 20a, and the side away from the host 20 may be a distal side 20b.

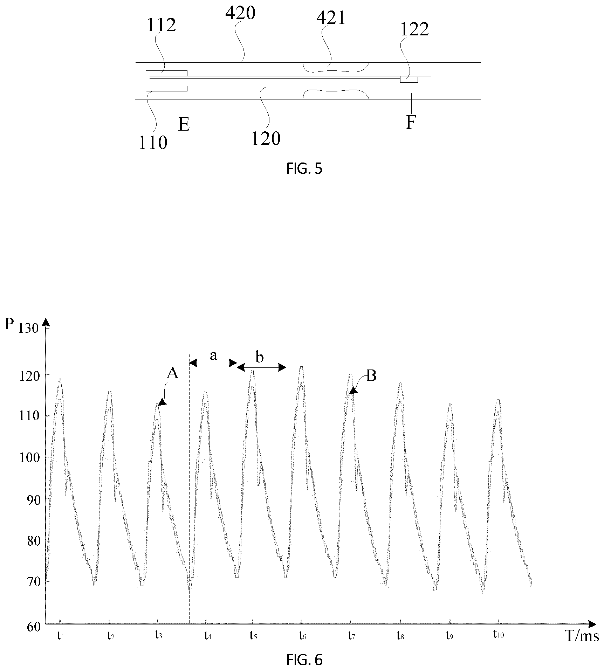

The system 1 for tracking the cardiac circulatory event by using the blood pressure involved by the present disclosure can measure the pressure in a blood vessel by using an interventional catheter technology to determine a pathological condition of a patient's blood vessel, such as a stenotic lesion 421 (referring to Fig. 5). The system 1 can be used to measure the pressure in the patient's blood vessel and process (for example, obtaining a pressure ratio described later, and then, obtaining an average value of corresponding pressure ratios in a target period), and thus, the pathological condition of the patient's blood vessel can be determined without the injection of the maximum congestion inducing drugs.

In some examples, the pressure measurement apparatus 10 may measure the pressure in the blood vessel and generate a pressure signal.

In some examples, as shown in Fig. 2, the pressure measurement apparatus 10 may include a first pressure measurement device and a second pressure measurement device. In some examples, the first pressure measurement device may have a first pressure sensor that can measure the pressure in the blood vessel and generate a pressure waveform (e.g. the first pressure signal described later). In some examples, the second pressure measurement device may have a second pressure sensor that can measure the pressure in the blood vessel and generate a pressure waveform (e.g. the second pressure signal described later). However, examples of the present disclosure are not limited thereto, in other examples, the pressure measurement apparatus may include a third pressure measurement apparatus, the third pressure measurement apparatus may have two pressure sensors, and the two pressure sensors may measure the pressure in the blood vessel at the same time and respectively generate pressure signals (e.g. a first pressure signal and a second pressure signal described later).

In some examples, the first pressure measurement device may measure the pressure in the blood vessel and generate a pressure waveform. In some examples, as shown in Fig. 2, the first pressure measurement device may be a guide catheter 110 which may have a pressure sensor 112 for measuring the intravascular pressure. In some examples, the guide catheter 110 may measure the pressure in the blood vessel (e.g. the pressure of a side close to the proximal side 20a in the blood vessel) and generate a pressure signal via the pressure sensor 112. However, examples of the present disclosure are not limited thereto, the first pressure measurement device may also be other apparatuses capable of measuring the pressure in the blood vessel.

In some examples, as shown in Fig. 2, the first pressure measurement device may be the guide catheter 110 which may be elongated tube shaped and may have an internal cavity 111. In some examples, the guide catheter 110 has a proximal side 110a close to the host 20 and a distal side 110b away from the host 20.

In some examples, the guide catheter 110 is provided with a pressure sensor 112. In some examples, as shown in Fig. 2, the pressure sensor 112 may be an invasive blood pressure sensor that may be directly connected to a port on the proximal side 110a of the guide catheter 110 so as to be provided on the guide catheter 110. For example, the pressure sensor 112 may be provided with a circular interface that matches the tubular structure of the guide catheter 110 and is used to be connected to the guide catheter 110. Therefore, the guide catheter 110 and the pressure sensor 112 can match each other more effectively, and the intravascular pressure can be measured in better way. In some examples, the pressure sensor 112 may sense the pressure generated by a fluid flowing from the distal side 110b to the inside of the internal cavity 111 of the guide catheter 110.

Specifically, the guide catheter 110 may be disposed in a blood vessel of a human body, and a port on the distal side 110b of the guide catheter 110 is disposed on a first preset position (e.g. the side close to the proximal side 20a in the blood vessel) in the blood vessel; the pressure sensor 112 may be provided in vitro and connected to the port on the proximal side 110a of the guide catheter 110, blood on the position of the proximal side in the blood vessel may flow into the guide catheter 110 and may flow from the distal side 110b of the guide catheter 110 to the proximal side 110a of the guide catheter 110 so as to be sensed by the pressure sensor 112, and therefore, the pressure sensor 112 may obtain the pressure in the internal cavity 111 of the guide catheter 110, i.e. the pressure on the first preset position in the blood vessel.

However, examples of the present disclosure are not limited thereto, in other examples, the pressure sensor 112 may be a capacitive pressure sensor, a resistive pressure sensor, a fiber optic pressure sensor, etc.; the pressure sensor 112 may be provided on the distal side 110b of the guide catheter 110, for example, the pressure sensor 112 may be provided on the outer wall of the guide catheter 110 close to the distal side 110b. For example, when the system 1 works, the guide catheter 110 may be disposed in a blood vessel of a human body, and the pressure sensor 112 may be disposed on the first preset position in the blood vessel. In this case, the pressure sensor 112 may directly sense the pressure on the first preset position in the blood vessel.

In some examples, the first preset position may be a side close to the proximal side 20a in the blood vessel, and therefore, the guide catheter 110 may acquire the pressure of the side close to the proximal side 20a in the blood vessel changing with a cardiac cycle at a certain sampling rate via the pressure sensor 112 and generate the pressure waveform.

In some examples, the first pressure measurement device may be connected to the host 20, and the pressure signals acquired by the first pressure measurement device may be transmitted to the host 20. For example, as shown in Fig. 2, the guide catheter 110 may be connected to the host 20 via a transmission wire. In this case, the pressure signals acquired by measurement of the pressure sensor 112 are transmitted to the host 20 via the transmission wire.

In some examples, the second pressure measurement device may measure the pressure in a blood vessel and generate a pressure waveform. In some examples, as shown in Fig. 2, the second pressure measurement device may be a blood pressure measurement catheter 120 which may have a pressure sensor 122 for measuring the pressure in the blood vessel. In some examples, the blood pressure measurement catheter 120 measures the pressure in the blood vessel (e.g. the pressure of a side away from the proximal side 20a in the blood vessel) and generates a pressure signal via the pressure sensor 122. However, examples of the present disclosure are not limited thereto, the second pressure measurement device may also be other apparatuses for measuring the pressure in the blood vessel, such as a medical guide wire with a pressure sensor.

In some examples, as shown in Fig. 2, the second pressure measurement device may be a blood pressure measurement catheter 120 which may be elongated tube shaped. In some examples, the blood pressure measurement catheter 120 may have a proximal side 120a close to the host 20 and a distal side 120b away from the host 20, and the blood pressure measurement catheter 120 may have an internal cavity 121.

In some examples, the blood pressure measurement catheter 120 may be provided with a pressure sensor 122, and the pressure sensor 122 may be provided on the distal side 120b of the blood pressure measurement catheter 120. In some examples, the pressure sensor 122 may be provided on the outer wall of the blood pressure measurement catheter 120. In some examples, the pressure sensor 122 may also be provided in the internal cavity 121 of the blood pressure measurement catheter 120, and the internal cavity 121 may have a window matching the pressure sensor 122. In some examples, when the system 1 works, the blood pressure measurement catheter 120 may be disposed in a blood vessel of a human body, and the pressure sensor 122 may be disposed on a second preset position in the blood vessel. In this case, the pressure sensor 122 can measure the pressure on the second preset position in the blood vessel.

In some examples, the second preset position may be a side away from the proximal side 20a in the blood vessel, and therefore, the blood pressure measurement catheter 120 may acquire the pressure of the side away from the proximal side 20a in the blood vessel changing with a cardiac cycle at a certain sampling rate via the pressure sensor 122 and generate the pressure waveform.

In some examples, the second pressure measurement device may be connected to the host 20. For example, as shown in Fig. 2, the blood pressure measurement catheter 120 may be connected to the host 20. The blood pressure measurement catheter 120 may be provided with a signal pathway 123 which may be provided in the internal cavity 121 of the blood pressure measurement catheter 120, and the signal pathway 123 may connect the pressure sensor 122 to the host 20. In this case, the pressure signals acquired by measurement of the pressure sensor 122 are transmitted to the host 20 via the signal pathway 123.

In some examples, the pressure sensor 122 may be a capacitive pressure sensor, a resistive pressure sensor, etc. In addition, the pressure sensor 122 may also be an MEMS pressure sensor. For example, the measurement range of the pressure sensor 122 is from about -50mmHg to about +300mmHg. According to the type of the pressure sensor 122, the signal pathway 123 may be a conductive medium such as an electric wire. In addition, in some embodiments, the signal pathway 123 may also be a wireless communication line, an infrared communication line, or an ultrasonic communication line.

In some examples, as shown in Fig. 2, if the first pressure measurement device is the guide catheter 110 and the second pressure measurement device is the blood pressure measurement catheter 120, the diameter of the internal cavity 111 of the guide catheter 110 may be greater than the external diameter of the blood pressure measurement catheter 120. In some examples, the blood pressure measurement catheter 120 may enter the internal cavity 111 from the proximal side 110a of the guide catheter 110 (see Fig. 2 and Fig. 3). In this case, when the system 1 works, the blood pressure measurement catheter 120 can be provided in the internal cavity 111, which facilitates the movement of the blood pressure measurement catheter 120 relative to the guide catheter 110, and the guide catheter 110 can be kept stationary.

In some examples, the guide catheter 110 and the blood pressure measurement catheter 120 may be respectively used as individual apparatuses to enter or exit the body of a patient. In this case, the medical care personnel may independently control the blood pressure measurement catheter 120 and the guide catheter 110. For example, when the system 1 works, the medical care personnel may control the blood pressure measurement catheter 120 to move relative to the guide catheter 110 (e.g. advance deep into or withdraw deep from a blood vessel in the body of the patient), and the guide catheter 120 can be kept stationary.

In some examples, the second pressure measurement device may be deeper into the body of the patient relative to the first pressure measurement device (see Fig. 5), and the second pressure sensor (e.g. the pressure sensor 122) may measure the intravascular pressure deeper into the human body (e.g. the side, away from the proximal side 20a, in the blood vessel) relative to the first pressure sensor (e.g. the pressure sensor 112).

Specifically, as shown in Fig. 2 and Fig. 3, if the first pressure measurement device is the guide catheter 110 and the second pressure measurement device is the blood pressure measurement catheter 120, during interventional therapy, the operating personnel such as medical care personnel firstly advance the guide wire from a certain position (e.g. the thigh artery) on the body of a patient (or sick person) to a certain position (e.g. the first preset position) in the blood vessel along the blood vessel; then, the guide catheter 110 is enabled to advance to the first preset position in the blood vessel along the guide wire so that the guide catheter 110 can measure the pressure on the first preset position; and next, the guide wire is withdrawn, Angiography is performed by using, for example, a contrast agent, and the medical guide wire (unshown) is further advanced to the second preset position in the blood vessel along the guide catheter 110. In this case, the blood pressure measurement catheter 120 is along the medical guide wire to make the internal cavity 121 of the catheter slide over the medical guide wire, moreover, the blood pressure measurement catheter 120 is moved by operating (e.g. pushing and/or pulling) the proximal side 120a (or an operating apparatus (unshown) connected to the proximal side 120a of the blood pressure measurement catheter 120) of the blood pressure measurement catheter 120 outside of the body of the patient, and the pressure sensor 122 may also be moved with the blood pressure measurement catheter 120 until the pressure sensor 122 is located on a preset position (e.g. the second preset position).

In some examples, the pressure sensor 112 and the pressure sensor 122 may measure the pressure in the blood vessel at the same time. For example, the pressure sensor 112 may measure the pressure on the first preset position to generate a pressure signal, and meanwhile, the pressure sensor 122 may measure the pressure on the second preset position to generate a pressure signal.

In some examples, the pressure measurement apparatus 10 may be provided with an X-ray-opaque development ring, and therefore, the pressure measurement apparatus 10 may obtain pressures on the preset positions (e.g. the first present position and the second preset position) in the blood vessel by measurement. In some examples, the pressure measurement apparatus 10 adopts a different type of apparatus for measuring the intravascular pressure, and therefore, it is also possible that the position where the development ring is provided is different. For example, if the first pressure measurement device is the guide catheter 110 and the pressure sensor 112 is the invasive blood pressure sensor, the development ring should be provided on the port on the distal side 110b of the guide catheter 110; and if the pressure sensor 112 is the capacitive pressure sensor, etc., the development ring should be provided on the guide catheter 110 and is located on one side of the pressure sensor 112, or the pressure sensor 112 is directly provided on the development ring. In some examples, if the second pressure measurement device is the blood pressure measurement catheter 120, the development ring should be provided on the blood pressure measurement catheter 120 and is located on one side of the pressure sensor 122, or the pressure sensor 122 is directly provided on the development ring. In this case, the medical care personnel may operate the pressure measurement apparatus 10 under the cooperation of angiography of an X-ray machine (unshown), and therefore, the pressure measurement apparatus 10 may obtain the intravascular pressure on the preset positions (e.g. the first present position and the second preset position).

In some examples, the side close to the proximal side 20a in the blood vessel may be a side of the coronary artery close to an aortic port (i.e. a proximal side of the coronary artery), and the side away from the proximal side 20a in the blood vessel may be a side of the coronary artery away from the aortic port (i.e. a distal side of the coronary artery). Therefore, pressures in blood vessels on two sides of the coronary artery can be obtained, and then, the pathological condition of the coronary artery can be determined. However, examples of the present disclosure are not limited thereto, in some examples, a blood vessel (also referred to as "target blood vessel") measured by the pressure measurement apparatus 10 may be voluntarily determined by the medical care personnel.

Fig. 4 is a blood vessel image of a preset part involved by an example of the present disclosure. Wherein Fig. 4(a) shows a blood vessel 410, a blood vessel 420, a blood vessel 430, a blood vessel 440, a blood vessel 450, and other small blood vessels. In examples of the present disclosure, a manner of processing the other small blood vessels may be the same as the manner of processing the blood vessel 410, the blood vessel 420, the blood vessel 430, the blood vessel 440, and the blood vessel 450, for the representation convenience, Fig. 4(b) shows blood vessels on preset parts without the other small blood vessels, wherein points A, B, C, D, E, and F are respectively corresponding positions in the corresponding blood vessels. For example, the blood vessel 410 may be the aorta, the blood vessel 420 may be the right coronary artery, the blood vessel 430 may be the left main coronary artery, the blood vessel 440 may be the anterior descending coronary artery, and the blood vessel 450 may be the circumflex artery. Fig. 5 is a sectional diagram showing that a system involved by an example of the present disclosure is applied to a blood vessel. The corresponding blood vessel shown in Fig. 5 is the blood vessel 420 in Fig. 4.

In some examples, the blood vessel image on the preset part can be obtained by angiography by using the contrast agent.

In some examples, the medical care personnel may perform intravascular pressure measurement on multiple blood vessels in the preset part, and therefore, the entire pathological condition of the preset part can be determined. In some examples, the medical care personnel may perform intravascular pressure measurement on single blood vessel in the preset part, and therefore, the pathological condition of that blood vessel can be determined.

In the present embodiment, the pressure measurement apparatus 10 may be configured to measure the pressure of the side, close to the proximal side 20a in the blood vessel at a certain sampling rate to generate a first pressure signal, and measure the pressure of the side away from the proximal side 20a in the blood vessel to generate a second pressure signal.

In some examples, the pressure measurement apparatus 10 may measure a specific position of the target blood vessel, i.e. the first preset position and the second preset position may be voluntarily determined by the medical care personnel.

In some examples, as shown in Fig. 5, with the blood vessel 420 as an example, point E in the blood vessel 420 may be used as the first preset position (i.e. the side close to the proximal side 20a in the blood vessel), point F in the blood vessel 420 may be used as the second preset position (i.e. the side away from the proximal side 20a in the blood vessel). Therefore, the pressure measurement apparatus 10 may be configured to measure the pressure of on point E in the blood vessel 420 at a certain sampling rate to generate a first pressure signal, and measure the pressure on point F in the blood vessel 420 to generate a second pressure signal. In some examples, the medical care personnel may select other blood vessels for measurement, such as the blood vessel 440 in which point B may be used as the first preset position and point C may be used as the second preset position and the blood vessel 450 in which point A may be used as the first preset position and point C may be used as the second preset position.

In some examples, the sampling rate of the pressure measurement apparatus 10 ranges from about 30Hz to 1.5KHz. For example, the pressure sensor 122 and the pressure sensor 112 may measure the intravascular pressure at the sampling rate of 30Hz, 50Hz, 100Hz, 200Hz, 250Hz, 300Hz, 400Hz, 500Hz, 600Hz, 700Hz, 1000Hz, 1100Hz, 1200Hz, 1300Hz, 1400Hz, or 1500Hz. Therefore, the pressure in the blood vessel can be measured more accurately.

In some examples, the pressure measurement apparatus 10 may measure pressures on different positions in the blood vessel at the same time and generate a plurality of pressure signals. For example, the pressure measurement apparatus 10 may include a first pressure measurement device and a second pressure measurement device, the first pressure measurement device may measure the pressure on the first preset position to generate a pressure signal, and meanwhile, the second pressure measurement device may measure the pressure on the second preset position to generate a pressure signal. Or the pressure measurement apparatus 10 may include a third pressure measurement device. The third pressure measurement device may include a plurality of pressure sensors located on different positions, and therefore, the pressure measurement apparatus 10 may measure the pressures on the different positions in the blood vessel at the same time to obtain the plurality of pressure signals.

In some examples, the first pressure signal and the second pressure signal may be obtained by simultaneous measurement of the pressure measurement apparatus 10. For example, the first pressure measurement device may measure the pressure of the side close to the proximal side 20a in the blood vessel to generate the first pressure signal, and the second pressure measurement device may measure the pressure of the side away from the proximal side 20a, in the blood vessel to generate the second pressure signal.

In some examples, the pressure signals obtained by measurement of the pressure measurement apparatus 10 may change with a cardiac cycle which may include a diastolic phase of diastole and a systolic phase of systole.

In some examples, the pressure measurement apparatus 10 may be configured to measure the pressure of the side close to the proximal side 20a in the blood vessel to generate the first pressure signal, and measure the pressure of the side away from the proximal side 20a in the blood vessel to generate the second pressure signal. For example, the pressure measurement apparatus 10 may include a first pressure measurement device and a second pressure measurement device, the first pressure measurement device may measure the pressure of the side close to the proximal side 20a in the blood vessel (i.e. the first preset position) to generate the first pressure signal, the second pressure measurement device may measure the pressure of the side away from the proximal side 20a in the blood vessel (i.e. the second preset position) to generate the second pressure signal, and the host 20 may receive the first pressure signal and the second pressure signal.

In some examples, before measuring the pressure on the second preset position to obtain the second pressure signal, the second pressure sensor may be firstly enabled to measure the pressure on the first preset position, i.e. the second pressure sensor and the first pressure sensor measure the pressure on the first preset position at the same time to generate a pressure signal, thereby performing correction. Specifically, before the second pressure sensor measures the pressure on the second preset position (e.g. point F of the blood vessel 420), the pressure sensor may be firstly corrected. The second pressure sensor may be firstly provided on the first preset position (e.g. point E of the blood vessel 420), that is, the second pressure sensor may measure the intravascular pressure on the first preset position. In this case, the first pressure sensor and the second pressure sensor measure the intravascular pressure on the first preset position at the same time to generate pressure signals, and the second pressure sensor can be verified according to the obtained pressure signals. In some examples, the pressure signals obtained by the first pressure sensor and the second pressure sensor may be transmitted to the host 20, and the host 20 may correct the second pressure sensor or the first pressure sensor (adjust the second pressure sensor or the first pressure sensor) according to the received pressure signals until there are no obvious differences between the pressure signal obtained by the second pressure sensor and the pressure signal obtained by the first pressure sensor. In some examples, the host 20 may display the received pressure signals on a display screen in a form of a waveform (described later), and the medical care personnel may correct the second pressure sensor or the first pressure sensor according to the obtained waveform until there are no obvious differences between the pressure signals obtained by the first pressure sensor and the second pressure sensor, that is, two corresponding changing curves displayed on the host 20 overlap.

In some examples, after the pressure sensors are corrected, the medical care personnel may move the second pressure sensor by operating the second pressure measurement device (e.g. pushing the proximal side 120a of the blood pressure measurement catheter 120 outside the body of the patient) or other devices, so that the second pressure sensor may measure the pressure on the second preset position. In this case, the first pressure sensor may measure the pressure on the first preset position to generate the first pressure signal, and the second pressure sensor may measure the pressure on the second preset position to generate the second pressure signal.

In some examples, after obtaining the second pressure signal by measurement, the second pressure sensor is moved to be capable of measuring the pressure on the first preset position, and then, the second pressure sensor is verified. Specifically, after the second pressure sensor obtains the second pressure signal by measurement, the medical care personnel may move the second pressure sensor by operating the second pressure measurement device (e.g. pulling the proximal side 120a of the blood pressure measurement catheter 120 outside the body of the patient) or other devices, so that the second pressure sensor may measure the pressure on the first preset position. In this case, the first pressure sensor and the second pressure sensor measure the intravascular pressure on the first preset position at the same time to generate the pressure signals, and the second pressure sensor may be verified according to the obtained pressure signals to judge the accuracy of the second pressure signal obtained by the second pressure sensor, for example, the second pressure sensor may drift during movement to cause an error of the second pressure signal obtained by measurement of the second pressure sensor.

In some examples, during the verification, the pressure signals obtained by the first pressure sensor and the second pressure sensor may be transmitted to the host 20, and the host 20 may verify the second pressure sensor according to the received pressure signals. For example, during the verification, if there are no obvious differences between the pressure signals obtained by the first pressure sensor and the second pressure sensor, it may be determined that the second pressure signal obtained by the second pressure sensor before can be normally used; and if there are obvious differences between the pressure signals obtained by the first pressure sensor and the second pressure sensor, it may be determined that the second pressure signal obtained by the second pressure sensor before cannot be normally used, and the accuracy of a first blood pressure value cannot be determined. In some examples, during the verification, the pressure signals obtained by the first pressure sensor and the second pressure sensor may be transmitted to the host 20, the host 20 may display the received pressure signals on a display screen in a form of a waveform (described later), and the medical care personnel may verify the second pressure sensor according to the obtained waveform to determine whether there are obvious differences between the pressure signals obtained by the first pressure sensor and the second pressure sensor, i.e. to determine whether the two corresponding changing curves displayed on the host 20 overlap. Therefore, the second pressure signal obtained by the second pressure sensor can be more accurate and can match the first pressure signal, which may facilitate the subsequent more effective determination for the pathological condition of a blood vessel to be measured.

Fig. 6 is a pressure waveform diagram showing a plurality of cardiac cycles involved by an example of the present disclosure. Curve A in Fig. 6 is a first pressure waveform, i.e. a curve that a pressure value corresponding to the first pressure signal changes with time; curve B is a second pressure waveform, i.e. a curve that a pressure value corresponding to the second pressure signal changes with time; interval a or interval b is a cardiac cycle respectively, wherein a vertical axis may be the magnitude of the pressure (i.e. the pressure value), and a horizontal axis may be a time axis.

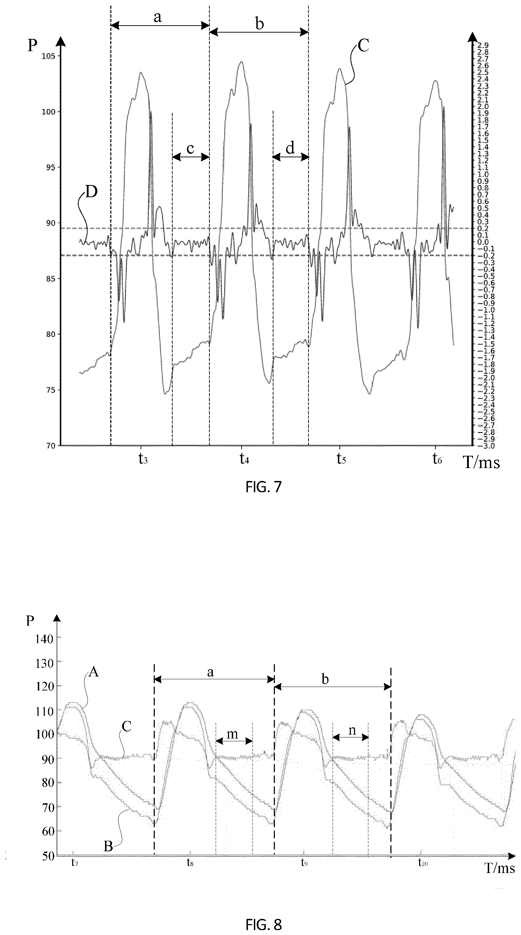

Fig. 7 is a pressure waveform diagram showing the plurality of cardiac cycles in Fig. 6 involved by an example of the present disclosure, wherein curve C in Fig. 7 is a target pressure waveform, curve D is a target derivative waveform, a pressure ratio shown by curve C is a hundred times as large as an actually calculated pressure ratio, and curve D is a curve formed by a derivative of curve C with respect to time. A vertical axis corresponding to curve C is the vertical axis at the left in Fig. 7, and a vertical axis corresponding to curve D is the vertical axis at the right in Fig. 7. The pressure ratio may be a ratio of a pressure value of the second pressure signal (see Fig. 6) to a pressure value corresponding to the pressure value of the second pressure signal of the first pressure signal (see Fig. 6), interval a or interval b is a cardiac cycle, interval c is a target period in interval a, and interval d is a target period in interval b.

Fig. 8 is a pressure waveform diagram showing the plurality of cardiac cycles in Fig. 6 involved by another example of the present disclosure. Curve A in Fig. 8 is a first pressure waveform, i.e. a curve that a pressure value corresponding to the first pressure signal changes with time; curve B is a second pressure waveform, i.e. a curve that a pressure value corresponding to the second pressure signal changes with time; and curve C is a target pressure waveform which is a curve that a pressure ratio changes with time (wherein the pressure ratio shown by curve C is a hundred times as large as an actually calculated pressure ratio), the pressure ratio may be a ratio of a pressure value of the second pressure signal to a pressure value corresponding to the pressure value of the second pressure signal of the first pressure signal, interval a or interval b is a cardiac cycle, interval m is a target period (described later) in interval a, and interval n is a target period in interval b. Wherein a vertical axis may be a pressure value or value, and a horizontal axis may be a time axis.

In some examples, when the system 1 works, the pressure measurement apparatus 10 measures the pressure in the blood vessel and generate a pressure signal which is then transmitted to the host 20, and the host 20 may display the received pressure signal on, for example, a display screen in a form of a waveform, i.e. a changing curve that the pressure corresponding to the pressure signal changes with time (see Fig. 6 to Fig. 8).

In some examples, the host 20 may process the received pressure signals.

In some examples, the host 20 may filter the received pressure signals.

In some examples, the pressure waveforms received by the host 20 at least include an entire cardiac cycle. In some examples, the host 20 may distinguish all cardiac cycles in the pressure waveforms on the basis of the obtained pressure waveforms.

In some examples, the host 20 may receive the first pressure signal and the second pressure signal, the host 20 may obtain the first pressure waveform (e.g. curve A in Fig. 6) on the basis of the first pressure signal, and the host 20 may obtain the second pressure waveform (e.g. curve B in Fig. 6) on the basis of the second pressure signal. In some examples, the first pressure waveform at least includes an entire cardiac cycle. In some examples, the second pressure waveform at least includes an entire cardiac cycle. In some examples, the host 20 may distinguish all cardiac cycles on the basis of the first pressure waveform and/or the second pressure waveform. Therefore, the first pressure waveform and the second pressure waveform can be obtained, and the host 20 can distinguish all the cardiac cycles on the basis of the first pressure waveform and/or the second pressure waveform.

In some examples, the host 20 may be configured to synchronize the received pressure signals (e.g. the pressure signals transmitted by the first pressure measurement device and the second pressure measurement device) so that the moments when peak values of all pressure waveforms received by the host 20 appear within the corresponding same cardiac cycle are the same. For example, when the second pressure sensor is corrected and verified, the host 20 may synchronize the received pressure signals measured by the first pressure sensor and the second pressure sensor.

In some examples, the host 20 may be configured to synchronize the first pressure waveform and the second pressure waveform so that a moment when a peak value of the first pressure waveform appears is the same as a moment when a peak value of the second pressure waveform appears in the cardiac cycle. Therefore, the first pressure waveform and the second pressure waveform may be synchronized, which facilitates the subsequent obtaining of an accurate pressure ratio. In some examples, the host 20 may determine the differential degree of variation tendencies of the first pressure waveform and the second pressure waveform by means of covariance, thereby determining whether the first pressure signal and the second pressure signal are synchronous or not. In some examples, the host 20 may synchronize the first pressure waveform and the second pressure waveform by means of the covariance and a cross-correlation function.

In some examples, the host 20 may preprocess the received pressure signals and may reject an invalid part in the pressure signals. In some examples, the host 20 may preprocess the pressure signals on the basis of the received pressure signals and a reference index. In some examples, the reference index may include a preset value range corresponding to the pressure signals, or a preset frequency range of a corresponding cardiac cycles, or a preset peak range of all cardiac cycles corresponding to the pressure signals, etc.

In some examples, the host 20 may preprocess the first pressure signal and the second pressure signal and may reject invalid signals in the first pressure signal and the second pressure signal. Therefore, more valid pressure signals can be obtained. In some examples, the host 20 may preprocess the first pressure signal and the second pressure signal on the basis of the comparison of first pressure signal, the second pressure signal and the reference index. In some examples, the invalid signals may include signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal. Therefore, the invalid signals in the first pressure signal and the second pressure signal can be rejected.

In some examples, intervals of the invalid signals rejected by the host 20 may be different according to the different reference indexes. In some examples, the minimum unit of each invalid signal rejected by the host 20 may be one cardiac cycle.

In some examples, the reference index may include the preset value range corresponding to the pressure signals. In some examples, if a value in the pressure signal does not appear in the preset value range, the cardiac cycle corresponding to the part may be entirely rejected. For example, the preset value range may be from 30 mmHg to 200mmHg, i.e. the reference index may include that values of both of the first pressure signal and the second pressure signal should be included within the range of from 30 mmHg to 200mmHg. Therefore, the parts in which the values are not within the range of from 30 mmHg to 200mmHg in the first pressure signal and the second pressure signal can be rejected. In some examples, the host 20 may entirely reject the cardiac cycles where the parts in which the values are not within the range of from 30 mmHg to 200mmHg in the first pressure signal and the second pressure signal are located.

In some examples, the reference index may include the preset peak value range of the peak value of the value corresponding to the pressure signal. In some examples, if the peak value in the pressure signal exceeds the preset peak value range, the cardiac cycle where the peak value is located may be entirely rejected. For example, the preset peak value range may be a range that the first percentage of the peak value of the first pressure signal should be greater than the peak value of the second pressure signal, and the first percentage may range from 105% to 130%, for example, the first percentage may be 105%, 110%, 115%, 120%, 125% or 130%. That is, the reference index may include the peak value that the first percentage of the peak value of the first pressure signal should be greater than the peak value of the second pressure signal. Therefore, it can be determined that the first percentage of the peak value of the first pressure signal in the first pressure signal and the second pressure signal is not smaller than the peak value of the second pressure signal, and the cardiac cycles corresponding to the peak value can be entirely rejected, for example, it is determined that the peak value of the second pressure signal in the first pressure signal and the second pressure signal is greater than 110% of the peak value of the first pressure signal, and the cardiac cycles corresponding to the peak value can be entirely rejected.

In some examples, the reference index may include the frequency range of the cardiac cycle corresponding to the pressure signal within a unit time. In some examples, if the occurrence frequency (i.e. Frequency) of the cardiac cycle corresponding to the pressure signal within a certain unit time is not within the frequency range, signals within the unit time are rejected. For example, the reference index may include that the frequency of the cardiac cycles corresponding to the first pressure signal and the second pressure signal should be from 40/min to 120/min, and therefore, signals within the period of time when the cardiac cycles corresponding to the first pressure signal and the second pressure signal every minute are not 40 to 120 can be rejected.

In some examples, the host 20 receives a plurality of corresponding pressure signals (e.g. the first pressure signal and the second pressure signal), if there is a part unsatisfying the reference index in any one pressure signal, the host 20 may reject the unsatiable part (e.g. a cardiac cycle corresponding to the unsatiable part) in the pressure signal, and the host 20 may also reject parts corresponding to the rejected part of the pressure signal in other pressure signals. For example, if there is a part unsatisfying the reference index in the first pressure signal, the host 20 may reject the unsatiable part (e.g. a cardiac cycle corresponding to the unsatiable part), and the host 20 may also reject a part corresponding to the rejected part of the first pressure signal in the second pressure signal.

In some examples, the host 20 may reject the part unsatisfying the reference index in the pressure signal, the other parts of the pressure signal may be normally used, and when the host 20 selects a plurality of continuous cardiac cycles, the cardiac cycles in front of and behind the rejected part can be used as the continuous cardiac cycles to be processed. In some examples, the host 20 may reject the unsatiable part in the pressure signal and make other parts form continuous waveforms. For example, if there is a part unsatisfying the reference index in the second and sixth cardiac cycles in the pressure signal, the host 20 may make other parts of the pressure signal form the continuous waveforms, and when selecting a plurality of continuous cardiac cycles, the host 20 may select the original first and third cardiac cycles as the continuous cardiac cycles.

However, examples of the present disclosure are not limited thereto, in some examples, the host 20 may reject the part unsatisfying the reference index in the pressure signal, if meeting the part unsatisfying the reference index when selecting a plurality of continuous cardiac cycles, the host 20 may reselect a plurality of continuous cardiac cycles from the cardiac cycles behind the part or reselect from other parts of the signal.

In the present embodiment, the pathological condition of the blood vessel may be evaluated according to a ratio of the minimum constant transmyocardial resistance measured when a target region of a blood vessel (e.g. the coronary artery) is normal to the minimum constant transmyocardial resistance measured when there is stenosis in the target region of the blood vessel. Specifically, according to a fluid mechanics formula, a blood flow volume Q, a pressure difference $\triangle p$ and blood flow resistance R in a blood vessel (e.g. the blood vessel may be a coronary artery) may satisfy

$$Q = \frac{\triangle p}{R} \quad (1),$$

the blood flow volume of the blood vessel may be obtained on the basis of the formula (1), and it satisfies

$$Q = \frac{P_a - P_v}{R_a + R_b} \frac{P_d - P_v}{R_b} \quad (2),$$

wherein $P_a$ represents the pressure of the side close to the proximal side 20a in the blood vessel, $P_d$ represents the pressure of the side away from the proximal side 20a in the blood vessel, $P_v$ represents the central venous pressure, $R_a$ represents vascular resistance, and $R_b$ represents microcirculation resistance. In a physiological status, the central venous pressure is equal to or nearly equal to 0,

$$\frac{R_b}{R_a + R_b} = \frac{P_d - P_v}{P_a - P_v} = \frac{P_d}{P_a} \quad (3)$$

may be obtained on the basis of the formula (2), and therefore, it can be known that the pathological condition of the blood vessel may be evaluated according to a ratio of the pressure of the side away from the proximal side 20a in the blood vessel to the pressure of the side close to the proximal side 20a in the blood vessel.

In some examples, the host 20 may process the received first pressure signal and second pressure signal, and the host 20 may obtain a ratio of a pressure value of the second pressure signal to a pressure value corresponding to the pressure value of the second pressure signal of the first pressure signal on the basis of the first pressure signal and the second pressure signal, thereby obtaining a pressure ratio. In some examples, the host 20 may generate a target pressure waveform (see curve C in Fig. 7) on the basis of the obtained pressure ratio.

In some examples, the host 20 may obtain a target derivative waveform (see curve D in Fig. 7) on the basis of the target pressure waveform. Specifically, the host 20 may obtain a derivative value of the pressure ratio with respect to time on the basis of the target pressure waveform, thereby obtaining a target derivative waveform with the derivative value changing with time.

In the present embodiment, a stationary phase of a signal $P_d/P_a$ may be sought by means of the target pressure waveform, which may also be a period tending to 0 in $d(P_d/P_a)/dt$, and therefore, the period (i.e. a target period described later) when the blood flow resistance is lowest and the most constant can be obtained. That is, the target period may be a period (i.e. the stationary phase) when the variation of the target pressure waveform tends to be gentle in the cardiac cycle, i.e. the target pressure waveform is less in amplitude variation within the period.

In some examples, the host 20 may determine a target period in any cardiac cycle on the basis of the target derivative waveform, wherein the target period may be a period in the cardiac cycle when a derivative value of the target derivative waveform changes within a preset value range. That is, the host 20 may determine the period in the cardiac cycle when the derivative value of the target derivative waveform changes within the preset value range from any cardiac cycle as the target period. However, examples of the present disclosure are not limited thereto, in other examples, the target period in any cardiac cycle may also be determined on the basis of the target derivative waveform (specifically described later).

In some examples, the target period may be continuous. In some examples, the target period may be a period in the cardiac cycle when the derivative value of the target derivative waveform changes within the preset value range, that is, the derivative value of the cardiac cycle in the target period may be within the preset value range.

In some examples, the host 20 may obtain the period when the derivative value tends to 0 from the target derivative waveform as the target period. In some examples, the target period may be a period when the derivative value of the target derivative waveform is within the preset value range (see interval c and interval d in Fig. 7). In some examples, the preset value range may be voluntarily set by the medical care personnel. In the examples involved by the present embodiment, the unit of the time axis is adjusted to be ms, the preset value range may be from -0.2/ms to 0.2/ms (see Fig. 7), however, examples of the present disclosure are not limited thereto, the preset value range may also be from -0.01/ms to 0.01/ms, from -0.02/ms to 0.02/ms, from -0.03/ms to 0.03/ms, from -0.04/ms to 0.04/ms, from -0.05/ms to 0.05/ms, from -0.06/ms to 0.06/ms, from -0.07/ms to 0.07/ms, from -0.08/ms to 0.08/ms, from -0.09/ms to 0.09/ms, from -0.10/ms to 0.10/ms, from -0.12/ms to 0.12/ms, from -0.15/ms to 0.15/ms, from -0.16/ms to 0.16/ms or from -0.18/ms to 0.18/ms, etc.

In some examples, the continuous time duration of the target period may be greater than a preset time value, for example, the derivative value of the target derivative waveform within the time duration corresponding to the target period may be within the preset value range (e.g. from -0.2/ms to 0.2/ms). In some examples, the preset time value may be selected within the range of from 0.1 to 0.5S, for example, the continuous time duration of the target period may be greater than 0.1S, 0.2S, 0.25S, 0.3S, 0.4S or 0.5S, etc. Therefore, a more valid target period can be obtained.

In some examples, the target period is within a diastolic phase of a corresponding cardiac cycle. Therefore, the target period within the diastolic phase can be obtained.

In some examples, the host 20 may also filter the target derivative waveform. That is, before the target period is determined, the host 20 may filter the target derivative waveform. Therefore, a more accurate target period can be obtained.

In other examples, the target period may be a period when the entire amplitude variation of the target pressure waveform of the cardiac cycle within the target period is smaller than a preset percentage (e.g. 10%), that is, a difference of the maximum value and minimum value of the target pressure waveform of the cardiac cycle within the target period may be smaller than a preset percentage (e.g. 10%) of the minimum value, or the difference of the maximum value and minimum value of the target pressure waveform of the cardiac cycle within the target period may be smaller than a preset percentage (e.g. 10%) of the maximum value.

In some examples, the target period may be a period when the entire variation of the target pressure waveform of the cardiac cycle within the target period is smaller than a preset percentage (see interval m or interval n in Fig. 8), the preset percentage may be voluntarily set by the medical care personnel and may be one within the range of 0-20%, for example, the preset percentage may be 1%, 2%, 2.5%, 2.6%, 3%, 4%, 5%, 5.5%, 6%, 7% , 7.5%, 8%, 9%, 10%, 11%, 11.5%, 12%, 13%, 14%, 15%, 15.5%, 16%, 17%, 18%, 18.5%, 19% or 20%, etc.

In some examples, the continuous time duration of the target period may be greater than a preset time value, for example, the entire amplitude variation of the pressure ratio of the target derivative waveform within the time duration corresponding to the target period may be smaller than a preset percentage (e.g. 10%). In some examples, the preset time value may be selected within the range of from 0.1 to 0.5S, for example, the continuous time duration of the target period may be greater than 0.1S, 0.2S, 0.25S, 0.3S, 0.4S or 0.5S, etc. Therefore, a more valid target period can be obtained.

In some examples, the host 20 may also filter the target pressure waveform obtained on the basis of the first pressure signal and the second pressure signal. That is, before the target period is determined, the host 20 may filter the target pressure waveform. Therefore, a more accurate target period can be obtained.

In some examples, the flat period in the target pressure waveform may be affected by filtration. For example, as filtration strength is different, and therefore, the entire variation of the target pressure waveform may also be different. In this case, the preset value range or the preset percentage may be affected by the filtration, and the preset value range or the preset percentage may be appropriately adjusted by the medical care personnel according to an actual filtration situation. For example, if the target derivative waveform is filtered on the basis of a 40-order FIR filter, the preset value range may be from -0.2/ms to 0.2/ms (see Fig. 7).

In some examples, the host 20 may calculate an average value of corresponding pressure ratios in the target period of the cardiac cycle. In some example, the host 20 or the medical care personnel may compare the obtained average value with a preset threshold to determine the pathological condition of a patient's blood vessel, and therefore, the pathological condition of the patient's blood vessel can be determined without the injection of the maximum congestion inducing drugs.

In some examples, the preset threshold may be obtained by collecting and processing the pressure in a blood vessel of a patient. In some examples, the preset threshold may also be obtained as a matter of previous experience and voluntarily set by the medical care personnel.

In some examples, the host 20 may obtain target periods respectively corresponding to a plurality of cardiac cycles on the basis of the target derivative waveform or the target pressure waveform,, and calculate average values of the corresponding pressure ratios in the target periods of all the cardiac cycles, thereby averaging the plurality of obtained average values to obtain a target average value. Therefore, the target average value can be obtained. In some examples, the plurality of cardiac cycles may be continuous, for example, the host 20 may obtain target periods respectively corresponding to five continuous cardiac cycles on the basis of the target derivative waveform or the target pressure waveform.

In some examples, the host 20 or the medical care personnel may compare the obtained average value with a preset threshold to determine the pathological condition of a patient's blood vessel, and therefore, the pathological condition of the patient's blood vessel can be determined without the injection of the maximum congestion inducing drugs.

In examples involved by the present embodiment, the pressure measurement apparatus 10 may be configured to measure the pressure of a side close to a proximal side in a blood vessel at a certain sampling rate to generate a first pressure signal, or measure the pressure of a side away from the proximal side in the blood vessel to generate a second pressure signal, and the host 20 may be connected to the pressure measurement apparatus and configured to receive the first pressure signal and the second pressure signal. The host 20 may obtain a ratio of a pressure value of the second pressure signal to a pressure value corresponding to the pressure value of the second pressure signal of the first pressure signal on the basis of the first pressure signal and the second pressure signal to obtain a pressure ratio and generate a target pressure waveform; the host 20 may obtain a derivative value of the pressure ratio with respect to time on the basis of the target pressure waveform, thereby obtaining a target derivative waveform; and the host 20 may determine a target period in any cardiac cycle on the basis of the target derivative waveform and obtain an average value of corresponding pressure ratios in the target period, wherein the target period may be a period in the cardiac cycle when a derivative value of the target derivative waveform changes within a preset value range. In this case, in the present embodiment, the pathological condition of a patient's blood vessel can be determined without the injection of the maximum congestion inducing drugs.

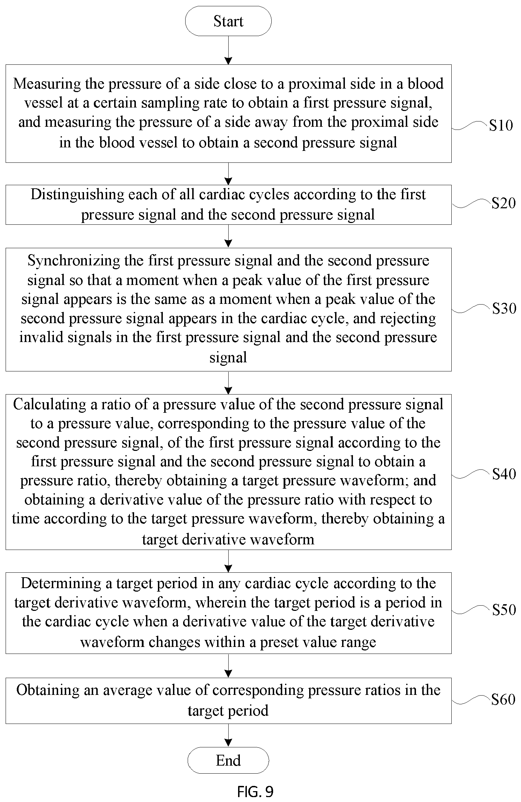

Fig. 9 is a schematic process diagram showing a method for tracking a cardiac circulatory event by using a blood pressure involved by an example of the present disclosure.

In the present embodiment, as shown in Fig. 9, the method for tracking the cardiac circulatory event by using the blood pressure may include the following steps: measuring the pressure of a side close to a proximal side in a blood vessel at a certain sampling rate to obtain a first pressure signal and measuring the pressure of a side away from the proximal side in the blood vessel to obtain a second pressure signal (step S10); distinguishing all cardiac cycles according to the first pressure signal and the second pressure signal (step S20); synchronizing the first pressure signal and the second pressure signal so that a moment when a peak value of the first pressure signal appears is the same as a moment when a peak value of the second pressure signal appears in the cardiac cycle, and rejecting invalid signals in the first pressure signal and the second pressure signal (step S30); calculating a ratio of a pressure value of the second pressure signal to a pressure value corresponding to the pressure value of the second pressure signal of the first pressure signal according to the first pressure signal and the second pressure signal to obtain a pressure ratio, thereby obtaining a target pressure waveform; and obtaining a derivative value of the pressure ratio with respect to time according to the target pressure waveform, thereby obtaining a target derivative waveform (step S40); determining a target period in any cardiac cycle according to the target derivative waveform, wherein the target period is a period, in the cardiac cycle, when a derivative value of the target derivative waveform changes within a preset value range (step S50); and obtaining an average value of corresponding pressure ratios in the target period (step S60).

In examples involved by the present implementation, the pressure of the side close to the proximal side in the blood vessel may be measured at a certain sampling rate to generate the first pressure signal, and the pressure of the side away from the proximal side in the blood vessel may be measured to generate the second pressure signal. All the cardiac cycles may be distinguished according to the first pressure signal and the second pressure signal; the first pressure signal and the second pressure signal may be synchronized, and the invalid signals in the first pressure signal and the second pressure signal may be rejected; the ratio of the pressure value of the second pressure signal to the pressure value corresponding to the pressure value of the second pressure signal of the first pressure signal may be calculated according to the first pressure signal and the second pressure signal to obtain the pressure ratio, thereby obtaining the target pressure waveform; the derivative value of the pressure ratio with respect to time may be obtained according to the target pressure waveform, thereby obtaining the target derivative waveform; the target period in any cardiac cycle may be determined according to the target derivative waveform, wherein the target period may be a period, in the cardiac cycle, when the derivative value of the target derivative waveform changes within the preset value range; and the average value of the corresponding pressure ratios in the target period may be obtained. In this case, according to the present disclosure, the pathological condition of a patient's blood vessel can be judged without the injection of the maximum congestion inducing drugs.

In the present embodiment, the first pressure signal, the second pressure signal, the target pressure waveform, the target derivative waveform and the target period in the method may be processed and obtained with reference to the above-mentioned first pressure signal, second pressure signal, target pressure waveform, target derivative waveform and target period. In some examples, processing in step S10 may be performed by utilizing an interventional catheter technology such as the pressure measurement apparatus 10. In some examples, processing in steps S20 to S60 may be performed by utilizing the host 20.

In step S10, as mentioned above, the pressure of the side close to the proximal side in the blood vessel may be measured at a certain sampling rate to generate the first pressure signal, and the pressure of the side away from the proximal side in the blood vessel may be measured to generate the second pressure signal.

In some examples, the pressure of the side close to the proximal side 20a in the blood vessel and the pressure of the side away from the proximal side 20a in the blood vessel may be respectively measured at a certain sampling rate by utilizing the interventional catheter technology such as the pressure measurement apparatus 10 to generate the first pressure signal and the second pressure signal. In some examples, the first pressure signal and the second pressure signal may be measured at the same time by the pressure measurement apparatus 10.

In some examples, the side close to the proximal side 20a in the blood vessel may be a side of the coronary artery close to an aortic port, and the side away from the proximal side 20a in the blood vessel may be a side of the coronary artery away from the aortic port. Therefore, pressures in blood vessels on two sides of the coronary artery can be obtained, and then, the pathological condition of the coronary artery can be determined.

In some examples, if the pressure measurement apparatus 10 includes the first pressure measurement device and the second pressure measurement device, the second pressure sensor may be corrected and verified.

In some examples, the sampling rate ranges from about 30Hz to 1.5KHz. For example, the first pressure sensor and the second pressure sensor may measure the intravascular pressure at the sampling rates 30Hz, 50Hz, 100Hz, 200Hz, 250Hz, 300Hz, 400Hz, 500Hz, 600Hz, 700Hz, 1000Hz, 1100Hz, 1200Hz, 1300Hz, 1400Hz, or 1500Hz. Therefore, the pressure in the blood vessel can be measured more accurately.

In step S20, as mentioned above, all cardiac cycles may be distinguished according to the first pressure signal and the second pressure signal.

In some examples, the host 20 may be utilized to receive the first pressure signal and the second pressure signal, and the host 20 may distinguish all the cardiac cycles according to the first pressure signal and the second pressure signal (the specific process may refer to the above-mentioned relevant descriptions).

In step S30, as mentioned above, the first pressure signal and the second pressure signal may be synchronized so that a moment when a peak value of the first pressure signal appears is the same as a moment when a peak value of the second pressure signal appears in the cardiac cycle, and invalid signals in the first pressure signal and the second pressure signal are rejected.

In some examples, the first pressure signal and the second pressure signal may be processed to a certain extent. In some examples, the first pressure signal and the second pressure signal may be filtered, and therefore, it facilitated to subsequently obtain a more accurate target pressure waveform.

In some examples, the first pressure signal and the second pressure signal may be synchronized so that a moment when a peak value of the first pressure signal appears is the same as a moment when a peak value of the second pressure signal appears in the cardiac cycle. Therefore, the first pressure signal and the second pressure signal may be synchronized, which facilitates the subsequent obtaining of an accurate pressure ratio. In some examples, the host 20 may determine the differential degree of variation tendencies of the first pressure waveform and the second pressure waveform by means of covariance, thereby determining whether the first pressure signal and the second pressure signal are synchronous or not. In some examples, the host 20 may synchronize the first pressure waveform and the second pressure waveform by means of the covariance and a cross-correlation function.

In some examples, the invalid signals in the first pressure signal and the second pressure signal may be rejected. Therefore, more valid pressure signals can be obtained. In some examples, the invalid signals may include signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal. Therefore, the invalid signals in the first pressure signal and the second pressure signal can be rejected.

In step S40, as mentioned above, the ratio of the pressure value of the second pressure signal to the pressure value corresponding to the pressure value of the second pressure signal of the first pressure signal may be calculated according to the first pressure signal and the second pressure signal to obtain the pressure ratio, thereby obtaining the target pressure waveform; and the derivative value of the pressure ratio with respect to time may be obtained according to the target pressure waveform, thereby obtaining the target derivative waveform.

In some examples, the host 20 may calculate the ratio of the pressure value of the second pressure signal to the pressure value corresponding to the pressure value of the second pressure signal of the first pressure signal according to the first pressure signal and the second pressure signal to obtain the pressure ratio. In some examples, the host 20 may obtain the target pressure waveform according to the obtained pressure ratio. In some examples, the host 20 may obtain the target derivative waveform according to the target pressure waveform. Specifically, the host 20 may obtain the derivative value of the pressure ratio with respect to time according to the target pressure waveform, thereby obtaining the target derivative waveform with the derivative value changing with time.

In step S50, as mentioned above, the target period in any cardiac cycle may be determined according to the target derivative waveform, wherein the target period may be a period in the cardiac cycle when a derivative value of the target derivative waveform changes within a preset value range.

In some examples, the host 20 may determine a target period in any cardiac cycle on the basis of the target derivative waveform, wherein the target period may be a period in the cardiac cycle when a derivative value of the target derivative waveform changes within a preset value range (e.g. from -0.2/ms to 0.2ms) (see interval c in Fig. 7). That is, the host 20 may determine a period in the cardiac cycle when the derivative value of the target derivative waveform changes within the preset value range, from any cardiac cycle as the target period.

In some examples, the target derivative waveform may be filtered. Therefore, it is facilitated to subsequently obtain a more accurate target period.

However, examples of the present disclosure are not limited thereto, in other examples, the host 20 may determine a target period in any cardiac cycle on the basis of the target pressure waveform, wherein the target period may be a period in the cardiac cycle when the variation of the target pressure waveform is smaller than a preset percentage (e.g. 10%) (see interval m or interval n in Fig. 8), that is, the host 20 may determine a period when the continuous variation of the target pressure waveform corresponding to the cardiac cycle is smaller than the preset percentage (e.g. 10%) from any cardiac cycle as the target period.

In some examples, In some examples, the target period may be continuous. In some examples, the continuous time duration of the target period may be greater than a preset time value, for example, the continuous time duration of the target period may be greater than 0.2s. In some examples, the preset time value may be selected within the range of from 0.1 to 0.5S. Therefore, a more valid target period can be obtained.

In some examples, the cardiac cycle may include a systolic phase of systole and a diastolic phase of diastole, and the target period may be within the diastolic phase. Therefore, the target period within the diastolic phase can be obtained.

In step S60, as mentioned above, the average value of corresponding pressure ratios in the target period can be obtained.

In some examples, the host 20 may calculate an average value of corresponding pressure ratios in the target period of the cardiac cycle. In some example, the target periods of a plurality of cardiac cycles may be obtained, the average values of the respective corresponding pressure ratios in the plurality of target periods of all the cardiac cycles may be obtained, and the plurality of average values may be averaged to obtain the target average value. In some examples, the host 20 or the medical care personnel may compare the obtained average value or target average value with a preset threshold to determine the pathological condition of a patient's blood vessel, and therefore, the pathological condition of the patient's blood vessel can be determined without the injection of the maximum congestion inducing drugs.

Although the present disclosure has been specifically described above with reference to the accompanying drawings and embodiments, it can be understood that the above-mentioned description does not limit the present disclosure in any form. Modifications and variations of the present disclosure, as required, may be made by those skilled in the art without departing from the true spirit and scope of the present disclosure, and shall fall into the scope of the present disclosure.

**Claims**

1. A system for tracking a cardiac circulatory event by using a blood pressure, **characterized by** comprising:

   a pressure measurement apparatus configured to measure the pressure of a side, close to a proximal side in a blood vessel at a certain sampling rate to generate a first pressure signal, and measure the pressure of a side away from the proximal side in the blood vessel to generate a second pressure signal; and
   a host connected to the pressure measurement apparatus and configured to receive the first pressure signal and the second pressure signal, the host distinguishing each of all cardiac cycles on the basis of the first pressure signal and the second pressure signal and calculating a ratio of a pressure value of the second pressure signal to a pressure value corresponding to the pressure value of the second pressure signal of the first pressure signal to obtain a pressure ratio and generate a target pressure waveform, the host obtaining a derivative value of the pressure ratio with respect to time on the basis of the target pressure waveform, thereby obtaining a target derivative waveform, and the host determining a target period in any cardiac cycle on the basis of the target derivative waveform, thereby obtaining an average value of corresponding pressure ratios in the target period, wherein the target period is a period in the cardiac cycle when a derivative value of the target derivative waveform changes within a preset value range.

2. The system for tracking the cardiac circulatory event by using the blood pressure according to claim 1, **characterized in that**, the host obtains a first pressure waveform at least comprising a complete cardiac cycle on the basis of the first pressure signal, the host obtains a second pressure waveform at least comprising a complete cardiac cycle on the basis of the second pressure signal, and the host distinguishes all the cardiac cycles on the basis of the first pressure waveform and/or the second pressure waveform.

3. The system for tracking the cardiac circulatory event by using the blood pressure according to claim 2, **characterized in that**, the host is configured to synchronize the first pressure waveform and the second pressure waveform so that a moment when a peak value of the first pressure waveform appears is the same as a moment when a peak value of the second pressure waveform appears in the cardiac cycle.

4. The system for tracking the cardiac circulatory event by using the blood pressure according to claim 1, **characterized in that**, the host obtains, on the basis of the target pressure waveform, target periods respectively corresponding to a plurality of cardiac cycles and calculates average values of corresponding pressure ratios in the target periods of all the cardiac cycles, thereby averaging the plurality of obtained average values to obtain a target average value.

5. The system for tracking the cardiac circulatory event by using the blood pressure according to claim 1, **characterized in that**, the cardiac cycle comprises a systolic phase of systole and a diastolic phase of diastole, and the target period is within the diastolic phase.

6. The system for tracking the cardiac circulatory event by using the blood pressure according to claim 1, **characterized in that**, the host preprocesses the first pressure signal and the second pressure signal to reject invalid signals in the first pressure signal and the second pressure signal, and the host filters the first pressure signal and the second pressure signal.

7. The system for tracking the cardiac circulatory event by using the blood pressure according to claim 6, **characterized in that**, the invalid signals comprise signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal.

8. The system for tracking the cardiac circulatory event by using the blood pressure according to claim 1, **characterized in that**, the target period is continuous, and the time duration of the target period is greater than a preset time value.

9. A method for tracking a cardiac circulatory event by using a blood pressure, **characterized by** comprising:

measuring the pressure of a side close to a proximal side in a blood vessel at a certain sampling rate to obtain a first pressure signal, and meanwhile, measuring the pressure of a side away from the proximal side in the blood vessel to obtain a second pressure signal; and distinguishing each of all cardiac cycles according to the first pressure signal and the second pressure signal; synchronizing the first pressure signal and the second pressure signal so that a moment when a peak value of the first pressure signal appears is the same as a moment when a peak value of the second pressure signal appears in the cardiac cycle, and rejecting invalid signals in the first pressure signal and the second pressure signal; calculating a ratio of a pressure value of the second pressure signal to a pressure value corresponding to the pressure value of the second pressure signal of the first pressure signal according to the first pressure signal and the second pressure signal to obtain a pressure ratio, thereby obtaining a target pressure waveform; obtaining a derivative value of the pressure ratio with respect to time according to the target pressure waveform, thereby obtaining a target derivative waveform; determining a target period in any cardiac cycle according to the target derivative waveform, thereby obtaining an average value of corresponding pressure ratios in the target period, wherein the target period is a period, in the cardiac cycle, when a derivative value of the target derivative waveform changes within a preset value range, wherein the invalid signals comprise signals of parts exceeding a preset value range in the pressure values corresponding to the first pressure signal and the second pressure signal, signals of parts with the occurrence frequency of the cardiac cycles exceeding a preset frequency range in the first pressure signal and the second pressure signal, and signals of parts exceeding a preset peak value range in peak values of the pressure values of all the cardiac cycles corresponding to the first pressure signal and the second pressure signal.

10. The method for tracking the cardiac circulatory event by using the blood pressure according to claim 9, **characterized in that**, the cardiac cycle comprises a systolic phase of systole and a diastolic phase of diastole; the target period is continuous and is within the diastolic phase, and the time duration of the target period is greater than a preset time value; and target periods respectively corresponding to a plurality of cardiac cycles are obtained according to the target pressure waveform, and average values of corresponding pressure ratios in the target periods of all the cardiac cycles are calculated, thereby averaging the plurality of obtained average values to obtain a target average value.

1

Host 20      Pressure measurement apparatus 10

FIG. 1

1

20a

10   123   120   122

Host 20

120a   121   120b

20b

112   110a   110   111   110b

FIG. 2

FIG. 3

(a)　(b)

FIG. 4

FIG. 5

FIG. 6

FIG. 7

FIG. 8

Start

Measuring the pressure of a side close to a proximal side in a blood vessel at a certain sampling rate to obtain a first pressure signal, and measuring the pressure of a side away from the proximal side in the blood vessel to obtain a second pressure signal — S10

Distinguishing each of all cardiac cycles according to the first pressure signal and the second pressure signal — S20

Synchronizing the first pressure signal and the second pressure signal so that a moment when a peak value of the first pressure signal appears is the same as a moment when a peak value of the second pressure signal appears in the cardiac cycle, and rejecting invalid signals in the first pressure signal and the second pressure signal — S30

Calculating a ratio of a pressure value of the second pressure signal to a pressure value, corresponding to the pressure value of the second pressure signal, of the first pressure signal according to the first pressure signal and the second pressure signal to obtain a pressure ratio, thereby obtaining a target pressure waveform; and obtaining a derivative value of the pressure ratio with respect to time according to the target pressure waveform, thereby obtaining a target derivative waveform — S40

Determining a target period in any cardiac cycle according to the target derivative waveform, wherein the target period is a period in the cardiac cycle when a derivative value of the target derivative waveform changes within a preset value range — S50

Obtaining an average value of corresponding pressure ratios in the target period — S60

End

FIG. 9

### INTERNATIONAL SEARCH REPORT

| International application No. |
|---|
| **PCT/CN2020/089285** |

| **A.** | **CLASSIFICATION OF SUBJECT MATTER** |
|---|---|

A61B 5/0215(2006.01)i;  A61M 25/01(2006.01)i

According to International Patent Classification (IPC) or to both national classification and IPC

| **B.** | **FIELDS SEARCHED** |
|---|---|

Minimum documentation searched (classification system followed by classification symbols)

A61B;A61M;G01F

Documentation searched other than minimum documentation to the extent that such documents are included in the fields searched

Electronic data base consulted during the international search (name of data base and, where practicable, search terms used)

WPI, EPODOC, CNKI, CNPAT: 北芯生命科技, 林佳燕, 心动周期, 血流储备分数, 循环, 预设, 范围, 导数, 时间, 血压, 压力, 第一, 第二, pressure, Fractional Flow Reserve, FFR, first, waveform, period, vessel, LIN Jiayan

| **C.** | **DOCUMENTS CONSIDERED TO BE RELEVANT** | | |
|---|---|---|---|
| Category* | Citation of document, with indication, where appropriate, of the relevant passages | | Relevant to claim No. |
| X | CN 107148239 A (KONINKLIJKE PHILIPS N.V. et al.) 08 September 2017 (2017-09-08) description, paragraphs [0034]-[0035], [0068]-[0071], figures 11-16 | | 1-10 |
| A | CN 107072568 A (MEDTRONIC VASCULAR, INC.) 18 August 2017 (2017-08-18) entire document | | 1-10 |
| A | CN 106264514 A (SHANGHAI ACOUSTIC LIFE SCIENCE CO., LTD.) 04 January 2017 (2017-01-04) entire document | | 1-10 |
| A | CN 104519791 A (WELLINQ MEDICAL B. V.) 15 April 2015 (2015-04-15) entire document | | 1-10 |
| A | CN 107736884 A (INSIGHT LIFETECH CO., LTD. (SHENZHEN)) 27 February 2018 (2018-02-27) entire document | | 1-10 |
| A | WO 2013028612 A2 (VOLCANO CORPORATION) 28 February 2013 (2013-02-28) entire document | | 1-10 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 January 2021** | **27 January 2021** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/089285**

| Patent document cited in search report | | | Publication date (day/month/year) | Patent family member(s) | | | Publication date (day/month/year) |
|---|---|---|---|---|---|---|---|
| CN | 107148239 | A | 08 September 2017 | JP | 2017530760 | A | 19 October 2017 |
| | | | | US | 2019083046 | A1 | 21 March 2019 |
| | | | | CN | 107148239 | B | 31 March 2020 |
| | | | | JP | 6596078 | B2 | 23 October 2019 |
| | | | | US | 2016073972 | A1 | 17 March 2016 |
| | | | | WO | 2016038493 | A1 | 17 March 2016 |
| | | | | EP | 3190958 | A1 | 19 July 2017 |
| | | | | US | 10130310 | B2 | 20 November 2018 |
| CN | 107072568 | A | 18 August 2017 | WO | 2016065227 | A2 | 28 April 2016 |
| | | | | EP | 3209199 | A2 | 30 August 2017 |
| | | | | EP | 3209199 | B1 | 24 April 2019 |
| CN | 106264514 | A | 04 January 2017 | | None | | |
| CN | 104519791 | A | 15 April 2015 | WO | 2014025255 | A1 | 13 February 2014 |
| | | | | EP | 2879575 | A1 | 10 June 2015 |
| | | | | JP | 2015529498 | A | 08 October 2015 |
| | | | | JP | 6235013 | B2 | 22 November 2017 |
| | | | | US | 2015223707 | A1 | 13 August 2015 |
| | | | | EP | 2879575 | B1 | 11 October 2017 |
| | | | | CN | 104519791 | B | 14 July 2017 |
| | | | | US | 10413201 | B2 | 17 September 2019 |
| CN | 107736884 | A | 27 February 2018 | CN | 107736884 | B | 12 October 2018 |
| WO | 2013028612 | A2 | 28 February 2013 | JP | 2014528774 | A | 30 October 2014 |
| | | | | EP | 2744400 | A2 | 25 June 2014 |
| | | | | RU | 2014110702 | A | 27 September 2015 |
| | | | | CA | 2846058 | A1 | 28 February 2013 |
| | | | | US | 10390768 | B2 | 27 August 2019 |
| | | | | US | 2015080749 | A1 | 19 March 2015 |
| | | | | JP | 6133864 | B2 | 24 May 2017 |
| | | | | EP | 2744400 | B1 | 24 May 2017 |

Form PCT/ISA/210 (patent family annex) (January 2015)